# EUROPEAN PATENT APPLICATION

(11) **EP 4 671 364 A1**
(43) Date of publication of application: **31.12.2025**
(21) Application number: 23924158.1
(22) Date of filing: 24.10.2023
(51) Int. Cl.: C12N 5/0775

(54) **CELL POPULATION CONTAINING CARDIAC STEM CELLS**

(30) Priority: 24.02.2023 JP 2023027405
(71) Applicant: Metcela Inc., Kawasaki-shi, Kanagawa 210-0821 (JP)
(72) Inventor: OKADA, Atsumasa, Tsuruoka-shi, Yamagata 997-0002 (JP)
(74) Representative: Jones, Nicholas Andrew
(86) International application number: PCT/JP2023/038232
(87) International publication number: WO 2024/176514

(57) **Abstract**

By expressing specific factors in cardiac stem cells, it was found that their ADM production ability, MMP1 production ability, and/or HGF production ability could be enhanced. Additionally, it was found that such cardiac stem cells exhibit excellent immune-regulatory and/or anti-inflammatory capabilities. Therefore, a cell population comprising such cardiac stem cells can be provided.

## Description

### Technical Field

The present invention relates to a cell population containing cardiac stem cells.

### Background Art

In recent years, research on stem cells derived from various organs has been advanced in the field of regenerative medicine, aiming to establish new treatments for various organ diseases by differentiating these stem cells into mature cells of the corresponding organs.

For example, Patent Document 1 discloses that research on stem cells derived from cardiac tissue has been conducted to realize regenerative treatment for cardiac diseases. It is disclosed that pluripotent stem cells derived from the heart can be used for the treatment of heart diseases by transplanting them. In Patent Document 1, the pluripotent stem cells are obtained by selecting and isolating cells that form spheres after being cultured in suspension from human heart tissue-derived cells. The cells disclosed in Patent Document 1 are characterized by the following surface antigen positive rates: c-kit: 0.2%, CD34: 0.8%, CD90: 65.3%, and CD105: 87.5%. Furthermore, these cells express genes such as Nkx-2.5, GATA4, ANP, α-cardiac actin, TNT, MLC2v, MLC2a, α-MHC (α-myosin heavy chain), and β-MHC (β-myosin heavy chain) after 21 days of differentiation into cardiomyocytes.

However, in this technical field, further development is being actively pursued in order to obtain stem cells with higher therapeutic efficacy and to minimize side effects that may occur during cell transplantation into patients. There is a demand for providing better stem cells.

### Prior Art Document

### Patent Documents

Patent Document 1: Domestic re-publication No. JP2006/093276

### Summary of the Invention

### Problems to be Solved by the Invention

The objective of the present invention is to provide a cell population containing improved cardiac stem cells.

### Means for Solving the Problems

The present inventor has studied to solve the above problem, and it is found that ADM production ability, MMP1 production ability and/or HGF production ability can be enhanced by expressing a specific factor in human stem cells derived from human, and it is found that such cardiac stem cells have excellent immunoregulatory ability and/or anti-inflammatory ability.

The present invention includes the following first aspect (Invention A):
[A1] A cell population comprising human-derived cardiac stem cells,
   wherein a proportion of cells expressing CD105 among all cells in the cell population is 70% or more,
   wherein the cell population does not express an αMHC gene, and
   wherein the cell population comprises cells with enhanced ADM production ability and/or enhanced MMP1 production ability.
[A2] The cell population according to [A1], wherein
   the enhanced ADM production ability is demonstrated by an ADM production amount of 1,300 pg/mL or more per 5.0 × 10⁵ cells after culturing the cell population for 5 days,
      and/or
   the enhanced MMP1 production ability is demonstrated by an MMP1 production amount of 100 pg/mL or more per 5.0 × 10⁵ cells after culturing the cell population for 5 days.
[A3] The cell population according to [A1] or [A2], wherein the cell population comprises cells with enhanced HGF production ability.
[A4] The cell population according to [A3], wherein the enhanced HGF production ability is demonstrated by an HGF production amount of 800 pg/mL or more per 10,000 cells after culturing the cell population for 3 days.
[A5] The cell population according to [A3], wherein the enhanced HGF production ability is demonstrated by an HGF production amount of 1,000 pg/mL or more per 10,000 cells after culturing the cell population for 3 days.
[A6] The cell population according to any one of [A1] to [A5], wherein proportions of cells expressing SIRPA, CD45, c-kit, and CD29 among all cells in the cell population are 60% or more, 5% or less, 30% or less, and 80% or more, respectively.
[A7] The cell population according to any one of [A1] to [A6], wherein proportion of cells expressing SIRPA, CD45, c-kit, and CD29 among all cells in the cell population are 60% or more, 5% or less, 10% or less, and 80% or more, respectively.
[A8] The cell population according to any of [A1] to [A7], wherein the cardiac stem cells are derived from human pediatric tissue.
[A9] A cell population comprising cardiac stem cells derived from human pediatric tissue,
   wherein a proportion of cells expressing CD105 among all cells in the cell population is 70% or more;
   wherein the cell population does not express the αMHC gene; and
   wherein proportions of cells expressing SIRPA, CD45, c-kit, and CD29 among all cells in the cell population are 60% or more, 5% or less, 30% or less, and 80% or more, respectively.
[A10] The cell population according to [A9], wherein the cell population comprises cells with enhanced HGF production ability.
[A11] The cell population according to [A10], wherein the enhanced HGF production ability is demonstrated by an HGF production amount of 800 pg/mL or more per 10,000 cells after culturing the cell population for 3 days.
[A12] The cell population according to [A10], wherein the enhanced HGF production ability is demonstrated by an HGF production amount of 1,000 pg/mL or more per 10,000 cells after culturing the cell population for 3 days.
[A13] The cell population according to any one of [A9] to [A12], wherein proportions of cells expressing SIRPA, CD45, c-kit, and CD29 among all cells in the cell population are 60% or more, 5% or less, 10% or less, and 80% or more, respectively.
[A14] A composition comprising the cell population according to any one of [A1] to [A13].

The present invention also includes the following second aspect (Invention B).
[B1] A pharmaceutical composition comprising the cell population according to any one of [A1] to [A13].
[B2] The pharmaceutical composition according to [B1] for improving cardiac function and/or inhibiting cardiac remodeling.
[B3] The pharmaceutical composition according to [B1] or [B2] for treating cardiac disease.
[B4] The pharmaceutical composition according to [B3], wherein the cardiac disease is ischemic heart disease, dilated cardiomyopathy, hypertrophic cardiomyopathy, or congenital heart disease including single ventricle disease.
[B5] The pharmaceutical composition according to any one of [B1] to [B4], wherein the composition is an injectable composition.
[B6] The pharmaceutical composition according to any one of [B1] to [B4], wherein the cell population is constructed as a cell tissue in a planar or three-dimensional form.

The present invention also includes the following third aspect (Invention C).
[C1] A method for improving cardiac function and/or suppressing cardiac remodeling in a subject, the method comprising administering or transplanting to the subject a cell population according to any of [A1] to [A13] or a composition according to [A14].
[C2] A method for treating cardiac disease in a subject, the method comprising administering or transplanting to the subject a cell population according to any of [A1] to [A13] or a composition according to [A14].
[C3] The method according to [C2], wherein the heart disease is ischemic heart disease, dilated cardiomyopathy, hypertrophic cardiomyopathy, or congenital heart disease including single ventricle disease.

The present invention also includes the following fourth aspect (Invention D).
[D1] Use of the cell population according to any of [A1] to [A13] or the composition according to [A14] as an injection for administration to cardiac tissue or its surrounding area for improving cardiac function and/or suppressing cardiac remodeling.
[D2] Use of the cell population according to any of [A1] to [A13] or the composition according to [A14] as an injection for administration to cardiac tissue or its surrounding area for the treatment of cardiac disease.
[D3] Use of the cell population according to any of [A1] to [A13] or the composition according to [A14] as a transplant material for transplantation into cardiac tissue or its surrounding area for improving cardiac function and/or suppressing cardiac remodeling.
[D4] Use of the cell population according to any one of [A1] to [A13] or the composition according to [A14] as a transplant material for transplantation into cardiac tissue or its surrounding area for the treatment of cardiac disease.

### Effects of the Invention

The present invention provides a cell population containing improved cardiac stem cells.

### Brief Description of the Drawings

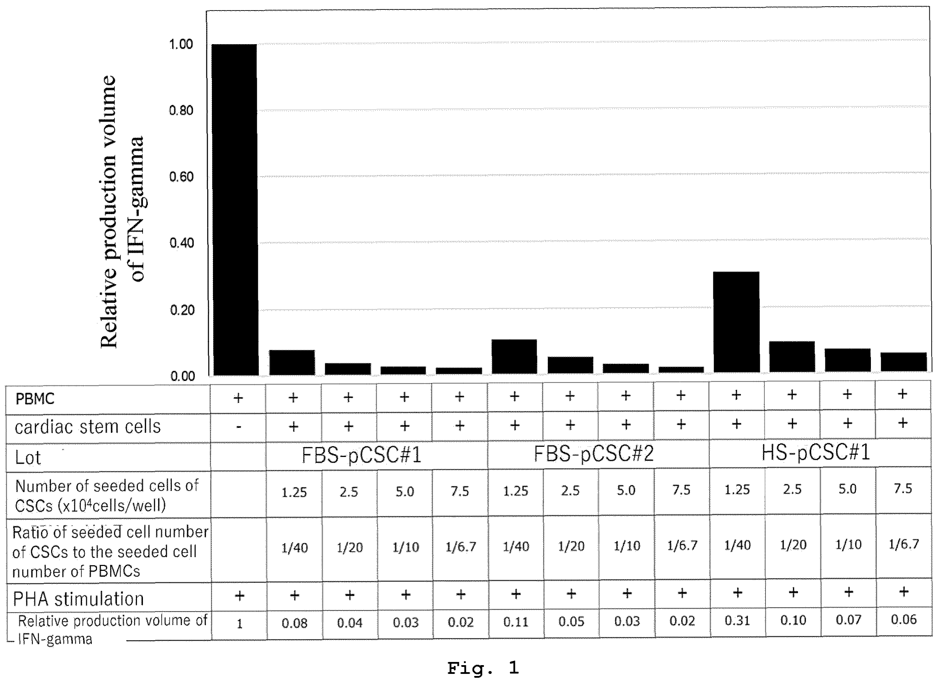

[Fig. 1] Fig. 1 shows a graph representing the relative production of IFN-gamma in each lot of cardiac stem cells.

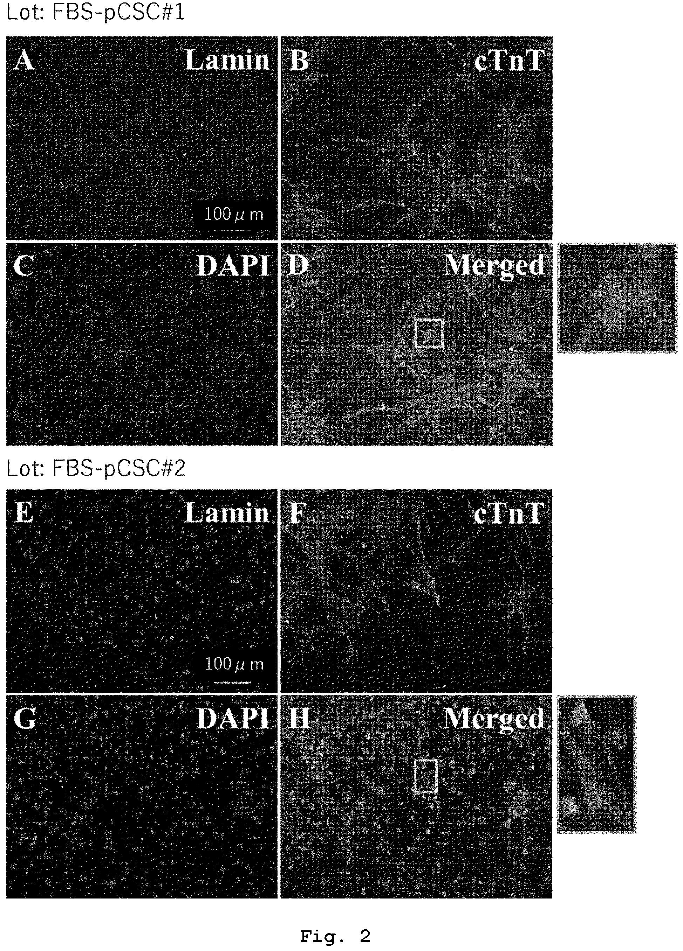

[Fig. 2] Fig. 2 shows a photograph representing the expression of cardiomyocyte-related marker (cTnT) in co-cultured mouse cardiomyocytes and human-derived cardiac stem cells after 5 days. Panels A to C show the staining images of Lamin, cTnT, and DAPI in cardiac stem cells from lot FBS-pCSC#1, and panel D shows the merged staining images of panels A to C. Panels E to G show the staining images of Lamin, cTnT, and DAPI in cardiac stem cells from lot FBS-pCSC#2, and panel H shows the merged staining images of panels E to G. The scale bar in panels A and E represents 100 µm. The magnified view of the rectangular area in panels D and H is shown on the right side of each panel.

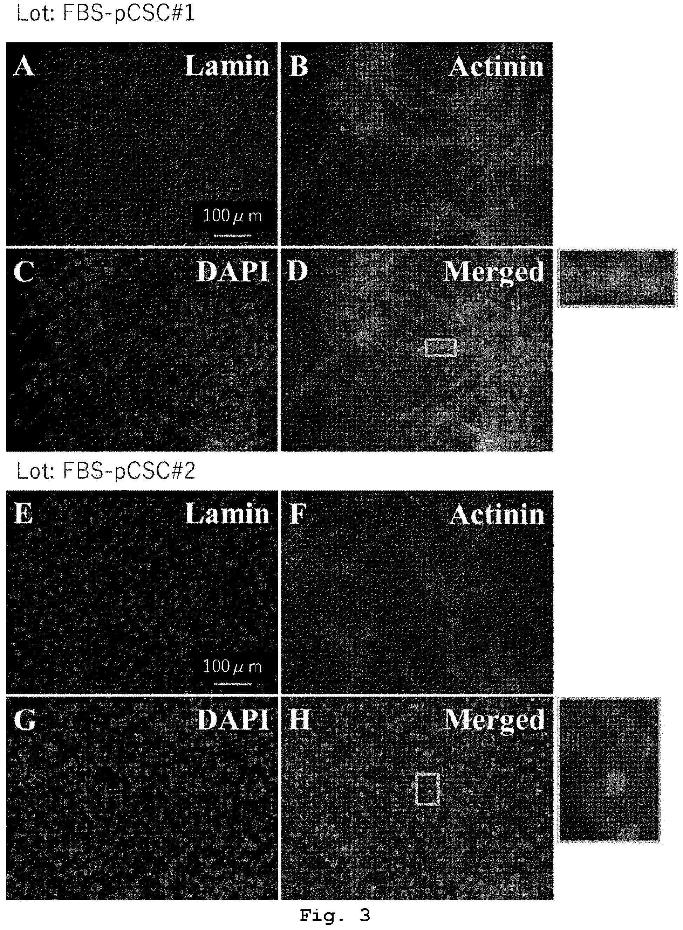

[Fig. 3] Fig. 3 shows a photograph representing the expression of cardiomyocyte-related marker (Actinin) in co-cultured mouse cardiomyocytes and human-derived cardiac stem cells after 5 days. Panels A to C show the staining images of Lamin, Actinin, and DAPI in cardiac stem cells from lot FBS-pCSC#1, and panel D shows the merged staining images of panels A to C. Panels E to G show the staining images of Lamin, Actinin, and DAPI in cardiac stem cells from lot HS-pCSC#2, and panel H shows the merged staining images of panels E to G. The scale bar in panels A and E represents 100 µm. The magnified view of areas enclosed by square frames in panels D and H are shown on the right side of each panel.

### Mode for Carrying Out the Invention

The present invention will be described in detail below with reference to specific embodiments. The present invention is, however, not limited to the illustrated specific embodiments presented.

One embodiment of the present invention is a cell population containing human-derived cardiac stem cells, wherein:
a proportion of cells expressing CD105 among all cells in the cell population is 70% or more; and
the cell population is one that does not express an αMHC gene.

It is preferred that the cell population contains cells with enhanced ADM production ability and/or enhanced MMP1 production ability.

The enhancement of ADM production ability may be evaluated, without particular limitation, based on the ADM production per 5.0 × 10⁵ cells after culturing the cell population for 5 days. The ADM production can be considered enhanced if it is 1,300 pg/mL or more, 2,000 pg/mL or more, 3,000 pg/mL, or more 5,000 pg/mL or more, 10,000 pg/mL or more, 20,000 pg/mL or more, 30,000 pg/mL or more, or 40,000 pg/mL or more.

The measurement of ADM production can be performed by methods known to those skilled in the art, such as by using the ELISA method.

The enhancement of MMP1 production ability may be evaluated, without particular limitation, based on the MMP1 production per 5.0 × 10⁵ cells after culturing the cell population for 5 days. The MMP1 production can be considered enhanced if it is 100 pg/mL or more, 500 pg/mL or more, 1,000 pg/mL or more, 2,000 pg/mL or more, 3,000 pg/mL or more, or 4,000 pg/mL or more.

The measurement of MMP1 production can be performed by methods known to those skilled in the art, such as by using the ELISA method.

The cardiac stem cells may be of either pediatric or adult origin, but are preferably of pediatric origin.

The cell population of this embodiment may include cells expressing CD105 as a cell surface antigen, and may further include cells expressing one or more cell surface antigens selected from the group consisting of CD90, SIRPA, and CD29. In addition to these cell surface antigens, the cell population may also include cells expressing any other cell surface antigen.

In the cell population of this embodiment, the proportion of cells expressing CD105 among all cells in the cell population is 70% or more, 80% or more, 90% or more, 95% or more, or 98% or more, based on the number of cells, and may be more than 70%, more than 80%, more than 90%, more than 95%, or more than 98%.

In the cell population of this embodiment, the proportion of cells expressing CD90 among all cells in the cell population may be 30% or more, 40% or more, 50% or more, 60% or more, 70% or more, 80% or more, 84% or more, 90% or more, 95% or more, or 98% or more, or may be more than 30%, more than 40%, more than 50%, more than 60%, more than 70%, more than 80%, more than 84%, more than 90%, more than 95%, or more than 98% based on the number of cells.

In the cell population of this embodiment, the proportion of cells expressing SIRPA among all cells in the cell population may be 60% or more, 70% or more, 79% or more, 80% or more, 90% or more, 95% or more, or 98% or more, based on the number of cells, and may be more than 60%, more than 70%, more than 79%, more than 80%, more than 90%, more than 95%, or more than 98%.

In the cell population of this embodiment, the proportion of cells expressing CD29 among all cells in the cell population may be 70% or more, 80% or more, 90% or more, 95% or more, or 98% or more based on the number of cells, or may be more than 70%, more than 80%, more than 90%, more than 95%, or more than 98%.

The cell population of this embodiment may include cells that do not express or express low levels of one or more cell surface antigens selected from the group consisting of CD45, CD34, CD31, and c-kit. The cell population may also include cells that do not express or express low levels of any cell surface antigen other than these cell surface antigens. Note that "not expressing" or "lowly expressing" refers to, for example, a percentage of cells based on the number of cells out of all cells in the cell population that is equal to or less than a predetermined percentage. This predetermined percentage may be a different value for each cell surface antigen, and can be expressed, for example, as follows:

In the cell population of this embodiment, the proportion of cells expressing CD45 among all cells in the cell population may be 10% or less, 5% or less, 4% or less, 3% or less, 2% or less, or 1% or less, based on the number of cells, and may be less than 10%, less than 5%, less than 4%, less than 3%, less than 2%, or less than 1%.

In the cell population of this embodiment, the proportion of cells expressing CD34 among all cells in the cell population may be 30% or less, 20% or less, 15% or less, 10% or less, 5% or less, or 1% or less, or may be less than 30%, less than 20%, less than 15%, less than 10%, less than 5%, or less than 1% based on the number of cells.

In the cell population of this embodiment, the proportion of cells expressing CD31 among all cells in the population is preferably 90% or less, 80% or less, 70% or less, 60% or less, 50% or less, 40% or less, 30% or less, 20% or less, 10% or less, 5% or less, or 1% or less, or less than 90%, less than 80%, less than 70%, less than 60%, less than 50%, less than 40%, less than 30%, less than 20%, less than 10%, less than 5%, or less than 1%, based on the cell count.

In the cell population of this embodiment, the proportion of cells expressing c-kit among all cells in the cell population may be 30% or less, 20% or less, 15% or less, 10% or less, 5% or less, or 1% or less, or may be less than 30%, less than 20%, less than 15%, less than 10%, less than 5%, or less than 1% based on the number of cells.

The cell population of this embodiment may include cells characterized by any combination of antigens expressed on the cell surface and antigens not expressed or expressed at low levels on the cell surface. For example, as long as the proportion of cells expressing CD105 among all cells in the population is 70% or more, the other cell surface antigens are not particularly limited, and the proportions of cells expressing SIRPA, CD45, c-kit, and CD29 among all cells in the population may be, for example, 60% or more, 5% or less, 10% or less, and 80% or more, respectively, or 70% or more, 5% or less, 10% or less, and 80% or more, respectively. Additionally, for instance, the proportion of cells expressing CD105 among all cells may be 90% or more, and the proportion of cells expressing CD45 and c-kit may be less than 10% and less than 10%, respectively. It is noted that SIRPA is a surface antigen specific to pluripotent stem cell-derived myocardial progenitor cells and cardiomyocytes.

The proportion of expression of cell surface antigens can be measured using methods known to those skilled in the art, such as flow cytometry. There are no particular limitations on the antibodies used for flow cytometry, so long as they can detect the respective cell surface antigens. They may be produced by a method known to those skilled in the art, or commercially available antibodies may be used.

The cell population of this embodiment does not express the αMHC (hereinafter also referred to as MYH6) gene.

The method for determining the expression of each gene is not particularly limited, but for example, the expression level of each gene in a population of human adult cardiac fibroblasts (standardized with beta-actin) can be determined as described below based on the relative expression level of each gene when the reference value is set to 1.0. The human adult cardiac fibroblasts may be derived from either the atrium or the ventricle.

In the cell population, not expressing the αMHC gene may mean not expressing it at all, or not expressing it substantially. Not expressing it substantially means, for example, that the expression level is below the detection sensitivity when measured by any measurement method. Alternatively, based on the gene expression level in a human adult cardiac fibroblast population, the expression level may be less than 1.5 times, less than 1.4 times, less than 1.3 times, less than 1.2 times, less than 1.1 times, less than 1.0 times, less than 0.9 times, less than 0.8 times, less than 0.7 times, less than 0.6 times, less than 0.5 times, less than 0.4 times, less than 0.3 times, less than 0.2 times, or less than 0.1 times.

In the cell population, the Gata4 gene may be expressed at 0.7 times or more, 0.8 times or more, 0.9 times or more, 1.0 times or more, 1.1 times or more, 1.2 times or more, 1.3 times or more, 1.4 times or more, 1.5 times or more, 1.6 times or more, 1.7 times or more, 1.8 times or more, or 1.9 times or more higher than the gene expression level in a human adult heart-derived fibroblast population, and it is preferable that the Gata4 gene is expressed at 1.0 times or more, 1.1 times or more, 1.2 times or more, 1.3 times or more, 1.4 times or more, 1.5 times or more, 1.6 times or more, 1.7 times or more, 1.8 times or more, or 1.9 times or more higher.

In the cell population, the Mef2C gene may be expressed at a level 1.0 times or more, 1.1 times or more, 1.2 times or more, 1.3 times or more, 1.4 times or more, 1.5 times or more, 1.6 times or more, 1.7 times or more, 1.8 times or more, 1.9 times or more, 2.0 times or more, 2.5 times or more, 3.0 times or more, or 3.5 times or more higher than the gene expression level in a human adult heart-derived fibroblast population.

The cell population may express one or more genes selected from the group consisting of the Tbx5 gene and the MYL2 gene. For example, in the cell population, the Tbx5 gene may be expressed at a level at 0.3 times or more, 0.4 times or more, 0.5 times or more, 0.6 times or more, 0.7 times or more, 0.8 times or more, 0.9 times or more, 1.0 times or more, 1.1 times or more, 1.2 times or more, 1.3 times or more, 1.4 times or more, 1.5 times or more, 1.6 times or more, 1.7 times or more, 1.8 times or more, 1.9 times or more, 2.0 times or more, 2.5 times or more, 3.0 times or more, or 3.5 times or more higher than the gene expression level in the human adult heart-derived fibroblast population, and it is preferably expressed 1.0 times or more, 1.1 times or more, 1.2 times or more, 1.3 times or more, 1.4 times or more, 1.5 times or more, 1.6 times or more, 1.7 times or more, 1.8 times or more, 1.9 times or more, 2.0 times or more, 2.5 times or more, 3.0 times or more, or 3.5 times higher. Further, for example, in the cell population, the MUL2 gene may be expressed at a level at 1.0 times or more, 1.1 times or more, 1.2 times or more, 1.3 times or more, 1.4 times or more, 1.5 times or more, 1.6 times or more, 1.7 times or more, 1.8 times or more, 1.9 times or more, 2.0 times or more, or 2.5 times or more higher than the gene expression level in the human adult heart-derived fibroblast population

Furthermore, the cell population may not express one or more genes selected from the group consisting of Nkx2.5, ELN, and TNN2. "Not expressing" means that it does not express at all or expresses it substantially not. "Substantially not expressing" means that the expression level is below the detection sensitivity using any measurement method. Alternatively, the gene expression level compared to the baseline in human adult heart-derived fibroblast populations may be less than 1.5 times, less than 1.4 times, less than 1.3 times, less than 1.2 times, less than 1.1 times, less than 1.0 times, less than 0.9 times, less than 0.8 times, less than 0.7 times, less than 0.6 times, less than 0.5 times, less than 0.4 times, less than 0.3 times, less than 0.2 times, or less than 0.1 times.

The cell population of this embodiment may contain cells characterized by any combination of expressed and unexpressed genes as described above.

Gene expression proportions can be measured using known methods, such as real-time PCR. The primers and PCR reagents used in real-time PCR are not particularly limited as long as they are capable of detecting the respective gene expressions. Primers or various PCR reagents may be produced by a method known to those skilled in the art, or commercially available primers or various PCR reagents may be used.

The cell population of this embodiment may contain cells characterized by any combination of the expressed cell surface antigens and expressed genes.

Furthermore, the cell population may contain cells expressing any intracellular antigen. The intracellular antigen may be measured by a method known to those skilled in the art, for example, by flow cytometry. The antibodies used for flow cytometry are not particularly limited as long as they can detect the respective cell surface antigens. They may be produced by a method known to those skilled in the art, or commercially available antibodies may be used.

The cell population of this embodiment may include cells with enhanced HGF production ability.

The HGF production ability may be enhanced by using FBS instead of human serum during cell culture.

The enhanced HGF production ability may be evaluated based on, but is not limited to, the amount of HGF produced per 10,000 cells after culturing the cell population for 3 days, and the amount of HGF production can be evaluated as enhanced when it is 800 pg/mL or more, 1,000 pg/mL or more, 1,200 pg/mL or more, 2,000 pg/mL or more, 3,000 pg/mL or more, 5,000 pg/mL or more, or 10,000 pg/mL or more, and it is preferable to evaluate the amount of HGF production as being enhanced if the amount of HGF produced is 1,000 pg/mL or more, 1,200 pg/mL or more, 2,000 pg/mL or more, 3,000 pg/mL or more, 5,000 pg/mL or more, or 10,000 pg/mL or more.

The amount of HGF produced can be measured by methods known to those skilled in the art, for example, by the ELISA method.

The cell population of the present embodiment may include cells with enhanced immune regulatory ability and/or anti-inflammatory ability.

The immunomodulatory and/or anti-inflammatory capabilities may be enhanced by using FBS instead of human serum during cell culture.

The enhanced immunomodulatory and/or anti-inflammatory capabilities may be, but are not limited to, enhanced ability to suppress IFNγ production by PBMCs, and may be evaluated based on the amount of IFNγ produced per 250,000 PBMCs after co-culturing the cell population and PBMCs for 3 days in the presence of PHA at a final concentration of 5 µg/ml. For example, the enhanced immunomodulatory and/or anti-inflammatory capabilities may be indicated by a lower amount of IFNγ produced per 250,000 PBMCs when the cell population and PBMCs are cultured under the above conditions, compared to the amount of IFNγ produced per 250,000 PBMCs after co-culturing a control cardiac stem cell population and PBMCs for 3 days under the same conditions. Here, for example, the control cardiac stem cell population may be a human pediatric cardiac stem cell population, in which the proportion of cells expressing CD105 among all cells in the control cardiac stem cell population is 70% or more, the cells do not express αMHC gene, and the expression of Gata4 gene is less than 1.0 times the gene expression level in a human adult cardiac fibroblast population. Furthermore, the enhanced immunoregulatory ability and/or anti-inflammatory ability may be an enhanced ability to suppress the proliferation of PBMCs or T cells, or an enhanced ability to suppress the production of TNFα.

The amount of IFNγ produced can be measured by methods known to those skilled in the art, for example, by the ELISA method.

The method for producing a cell population according to this embodiment is not particularly limited as long as the proportion of cells expressing CD105 among all cells in the cell population is at least 70% and the cell population has the characteristic of not expressing the αMHC gene, but may include, for example, the following cardiac stem cell isolation process and each culture process (primary culture process, expansion culture process).

### (Isolation of cardiac stem cells)

There is no particular limitation as long as cardiac stem cells can be isolated, but for example, cardiac stem cells can be isolated by the following method.

Human cardiac tissue may be minced to a desired size and subjected to cell dispersion treatment. The cell dispersion treatment may be performed using a tissue dispersion reagent such as collagenase. The tissue dispersion reagent may contain a DNase I solution. The liquid containing the cells after the cell dispersion treatment may be collected in a centrifuge tube and incubated at, for example, 37°C.

The culture medium used for the isolation of cardiac stem cells and each of the following culture steps is not particularly limited as long as it can be used for cardiac stem cell culture and for example, DMEM/F12 or the like can be used.

In the case of using a serum medium, it is preferable to add fetal bovine serum. On the other hand, as a serum medium for culturing control cardiac stem cells, it is preferable to add pooled human adult serum. The serum concentration in each medium is not particularly limited, but may be, for example, 1 to 20% or 5 to 15%.

Any other components may be added to the medium, for example, growth factors such as bFGF and EGF, antibiotics such as penicillin and streptomycin, cytokines, and the like.

The type, concentration, and reaction time of each reagent; the rotation speed, temperature, and time of centrifugation; and the incubation temperature and time for each treatment can be adjusted as necessary.

During the isolation process, washing treatments or other optional processes may be performed between each step.

### (Primary Culture)

The following culture process are not particularly limited as long as the isolated cardiac stem cells can be primary cultured or subcultured. After incubation in a serum medium during the isolation process, the supernatant containing cells is collected to obtain isolated cardiac stem cells.

The isolated cardiac stem cells are cultured in a CO₂ incubator (for example, 37°C, 5% CO₂). The culture medium may be replaced with a serum medium as necessary, and culturing can continue for more than one day. The culture period can be appropriately adjusted according to the intended purpose, such as when the cultured cells reach the desired cell number or acquire the desired function.

### (Expansion Culture)

After primary culture, the culture supernatant is removed. Any cell detachment treatment, such as trypsin/EDTA solution treatment, may be performed. The cells are collected using a serum medium, seeded into a new culture vessel, and expansion culture is started in a CO₂ incubator (for example, 37°C, 5% CO₂). The serum medium may be replaced every 1-7 days. The culture period can be appropriately set depending on the purpose, such as when the cultured cells reach the desired cell number or acquire the desired function.

In the isolation process, washing steps may be optionally performed between each step.

Furthermore, the obtained cardiac stem cells may be cryopreserved as desired. In this case, they can be thawed by any method upon use. The above-mentioned culture methods can be used for culture after thawing and subsequent subculture.

Here, the cardiac stem cells described in Patent Literature 1 are cells obtained by selecting and isolating cells that form spheres after floating culture of human heart tissue-derived cells. As a result, the cell population containing the cardiac stem cells described in Patent Literature 1 has different characteristics compared to the cell population of this embodiment. In fact, the pluripotent stem cells disclosed in Patent Literature 1 have a positive rate for cell surface antigens as follows: c-kit: 0.2%, CD34: 0.8%, CD90: 65.3%, CD105: 87.5%. Moreover, the cells described in Patent Literature 1 show the expression of genes such as Nkx-2.5, GATA4, ANP, α-ca-actin, TNT, MLC2v, MLC2a, α-MHC (α-myosin heavy chain), and β-MHC (β-myosin heavy chain) after 21 days of differentiation induction.

In contrast, the method of producing the cell population according to this embodiment involves adhesion culture without forming floating spheres. Additionally, the production method of the cell population in this embodiment does not involve selecting and isolating floating cells. As a result, the proportion of CD105-expressing cells in the total population of this embodiment is 70% or more, and it does not express the MYH6 (αMHC) gene, which is a characteristic feature.

The cell population of this embodiment may include cells t capable of differentiating into cardiomyocytes, i.e., cardiac progenitor cells.

The ability to differentiate into cardiomyocytes can be evaluated by inducing differentiation of the cell population into cardiomyocytes. Specifically, the cells after differentiation induction may be confirmed by expressing cell surface antigens specific to cardiomyocytes, such as cTnT, actinin, etc. Alternatively, the cells after differentiation induction may be confirmed by having morphological characteristics of cardiomyocytes, such as forming sarcomeres. Alternatively, the cells after differentiation induction may be confirmed by having functional characteristics of cardiomyocytes, such as pulsating.

Cell surface antigens, morphological characteristics, and functional characteristics can all be measured using methods known to those skilled in the art. For example, cells after differentiation can be reacted with primary antibodies recognizing cardiomyocyte-specific cell surface antigens, and a secondary antibody labeled with a fluorescent marker can be used for fluorescence microscopy.

Differentiation into cardiomyocytes may be considered to have occurred when at least one of the cell surface antigen, morphological characteristics, or functional characteristics shows features of cardiomyocytes. However, it is preferred to evaluate differentiation when all of these characteristics are present.

The method for inducing differentiation of human-derived cardiac stem cells into cardiomyocytes is not particularly limited, but it can be performed by treating human-derived cardiac stem cells with 5-Azacytidine and then co-culturing them with mammalian-derived cardiomyocytes.

The medium used for the 5-Azacytidine treatment is not particularly limited as long as it is a medium suitable for culturing cardiac stem cells or cardiomyocytes, and for example, the medium described in the above section on "Isolation of Cardiac Stem Cells" can be used as a base. Factors that initiate differentiation into cardiomyocytes, such as 5-Azacytidine, may be added to this medium. The concentration of 5-Azacytidine is not particularly limited, and may be, for example, 1 to 20 µM, or 5 to 15 µM.

The cells can be cultured for about 24 hours in a CO₂ incubator (for example, 37°C, 5% CO₂) using the medium containing 5-Azacytidine.

Co-culturing with mammalian-derived cardiomyocytes is not particularly limited, and examples thereof include co-culturing with mouse cardiomyocytes or human cardiomyocytes.

Pre-cultured mammalian cardiomyocytes can be mixed with the 5-Azacytidine-treated human cardiac stem cell suspension and cultured in a CO₂ incubator (for example, 37°C, 5% CO₂) to induce differentiation of the cardiac stem cells into cardiomyocytes.

The medium for co-culturing human-derived cardiac stem cells and mammalian cardiomyocytes is not particularly limited as long as it does not inhibit differentiation into cardiomyocytes. For example, the medium described in the section on "Isolation of Cardiac Stem Cells" or DMEM-GlutaMAX can be used, and these media can be mixed in any desired ratio.

It is preferable to add fetal bovine serum (FBS) to the medium, and the serum concentration in the medium may be for example, 1 to 20%, or 5 to 15%.

Any other components may be added to the medium, for example, growth factors such as bFGF and EGF, antibiotics such as penicillin and streptomycin solutions, cytokines, and dyes such as phenol red.

The type, concentration, and reaction time of each reagent, as well as the incubation temperature and time, can be adjusted as needed.

The culture period can be appropriately set as long as the cells differentiate into cardiomyocytes. For example, it may be 1 day or more, 2 days or more, 3 days or more, 4 days or more, 5 days or more, 6 days or more, or 7 days or more, and may be 14 days or less, or 10 days or less.

### (Composition)

Another embodiment of the present invention is a composition comprising the above-mentioned cell population, which may be used as a pharmaceutical composition.
the composition can be produced by subjecting the above-mentioned cardiac stem cells to any cell detachment treatment, such as treatment with trypsin/EDTA solution, and then suspending the desired number of cells in a commercially available cell preservation solution. After that, the composition may be refrigerated at 2 to 8°C as necessary. Furthermore, freezing may be performed as necessary.

The desired cell number of cardiac stem cells contained in the composition of this embodiment may be, for example, 1.0 × 10² cells/mL or more, 1.0 × 10³ cells/mL or more, 1.0 × 10⁴ cells/mL or more, 1.0 × 10⁵ cells/mL or more, 5.0 × 10⁵ cells/mL or more, 1.0 × 10⁶ cells/mL or more, 5.0 × 10⁶ cells/mL or more, 1.0 × 10⁷ cells/mL or more, 1.0 × 10⁸ cells/mL or more, or may be 1.0 × 10¹² cells/mL or less, 1.0 × 10¹¹ cells/mL or less, 1.0 × 10¹⁰ cells/mL or less, 1.0 × 10⁹ cells/mL or less. Furthermore, these upper and lower limits can be combined to form a certain range, for example, 1.0×10 ² cells/mL or more and 1.0×10 ¹¹ cells/mL or less, 1.0×10 ⁴ cells/mL or more and 1.0×10 ¹⁰ cells/mL or less, or 1.0×10 ⁶ cells/mL or more and 1.0×10 ⁸ cells/mL or less. The number of cells may be appropriately adjusted depending on the type and severity of the disease, and the age, height, weight, etc. of the patient.

The dosage of the composition containing the above-mentioned number of cardiac stem cells may be, for example, 0.1 mL or more, 0.5 mL or more, 1 mL or more, 2 mL or more, 3 mL or more, 4 mL or more, 5 mL or more, 10 mL or more, and may also be 20 mL or less, 15 mL or less. These upper and lower limits may also be combined to form a certain range, for example, 0.1 mL or more and 20 mL or less, or 0.5 mL or more and 15 mL or less. The dosage may be adjusted as appropriate depending on the type and severity of the disease, and the age, height, weight, etc. of the patient.

The method of administering or transplanting the composition of this embodiment to a living body is not particularly limited, but for example, it can be administered by injection. By directly injecting or transplanting the composition into the cardiac tissue and/or its surrounding area, it is possible to treat cardiac diseases, improve heart function, and suppress heart remodeling as described below. The composition may be administered to veins, arteries, lymphatic vessels, lymph nodes, or bone marrow. Administration to veins, arteries, or lymphatic vessels may be done by injection into the vascular system or by injection through a catheter, or using other known methods.

The method of injection is not particularly limited, and known injection techniques such as needle-based injections or needle-free injections can be applied, and the catheter method used for injection is also not particularly limited, and known methods can be applied.

The composition of this embodiment may be the above-mentioned cardiac stem cells cultured to form a cell sheet. The cell sheet may be a single layer or multiple layers. Culturing the cells to form a cell sheet can be performed using methods known to those skilled in the art. In this case, the composition of this embodiment can be administered or transplanted to a living body by, for example, attaching the composition cultured to form a cell sheet to the surface of the heart, which makes it possible to treat cardiac diseases, improve cardiac function, inhibit cardiac remodeling, etc., as described below.

The serum added to the culture medium may be fetal bovine serum or human adult pooled serum, and the concentration of serum in the medium is not particularly limited, but may be, for example, 1-20% or 5-15%.

The medium may further contain other physiologically acceptable components, such as physiological saline, buffer solutions, cell preservation solutions, cryopreservation solutions, and the like.

Additionally, other ingredients having medicinal properties may be included.

By administering or transplanting the composition of the present embodiment to a patient, the therapeutic effect of the cardiac stem cells contained in the composition can be exerted. The therapeutic effects are not particularly limited, but may include, for example, the regeneration of cardiac tissue by differentiation of the administered cardiac stem cells into cardiomyocytes in the cardiac tissue. Furthermore, the composition may have a high production ability of HGF, leading to enhanced cardiac protective effects, anti-apoptotic effects, angiogenic effects, and promotion of cardiomyocyte proliferation compared to conventional cardiac stem cells. Additionally, the composition may have a high immune-modulatory and anti-inflammatory effect, which could reduce side effects during administration in patients and lower inflammation in the cardiac tissue after surgery. In other words, since the cardiac stem cells contained in the composition have the ability to differentiate into cardiomyocytes and produce HGF, which is effective for heart function recovery, administering such a composition to a patient may treat heart diseases that require regeneration of cardiac tissue while minimizing side effects and improving heart function in patients with heart failure and suppressing heart remodeling.

Examples of cardiac diseases to be treated include ischemic heart disease, dilated cardiomyopathy, hypertrophic cardiomyopathy, or congenital heart diseases including single ventricle.

Moreover, improvement of heart function is not particularly limited, but may be assessed, for example, by suppressing left ventricular systolic dysfunction, i.e., by preventing worsening of heart function. Specifically, a decrease in left ventricular ejection fraction (EF) and/or left ventricular fractional shortening (%FS) observed via echocardiography can be used as an indicator of suppressed heart function deterioration.

Furthermore, the suppression of cardiac remodeling is not particularly limited, but can be assessed by observing the inhibition of left ventricular end-diastolic diameter (LVIDd) and/or left ventricular end-systolic diameter (LVIDs) enlargement via echocardiography, indicating suppression of heart remodeling.

The cells contained in the composition may be autologous cells, for example, cells derived from the cardiac tissue of a patient suffering from heart disease. The cells may be non-autologous, for example, cells derived from donor cardiac tissue. From the viewpoint of suppressing side effects during administration, autologous cells are preferable.

### <Treatment Method>

Another embodiment of the present invention may be a method for improving heart function and/or inhibiting heart remodeling by administering or transplanting the above-mentioned cell population or composition containing it to the target heart tissue and/or its surrounding area.

Furthermore, it may be a method for treating heart disease by administering or transplanting the cell population or composition containing it to the target heart tissue and/or its surrounding area.

### <Use as Injection or Transplantation Material>

Another embodiment of the present invention is the use of the cell population or composition containing it as an injectable formulation for improving heart function and/or inhibiting heart remodeling by administering it to the heart tissue and/or its surrounding area. The use of the cell population or composition comprising it as an injectable formulation for treating heart disease by administering it to the heart tissue and/or its surrounding area.

Another embodiment of the present invention is the use of the cell population or composition containing it as a transplant material for heart function improvement and/or inhibiting heart remodeling by transplanting it to the heart tissue and/or its surrounding area. The use of the cell population or composition containing it as a transplant material for treating heart disease by transplanting it to the heart tissue and/or its surrounding area.

### Examples

The present invention will be described in more detail below with reference to Examples, which do not limit the scope of the present invention.

### [Example 1: Method for Culturing Cardiac Stem Cells]

### <Preparation of Serum-Free Medium and Serum Medium>

Human bFGF (Fibroblast Growth Factor (basic), Human, recombinant) and human EGF (Epidermal Growth Factor, Human, recombinant) were each dissolved in injectable water to prepare bFGF and EGF solutions. The bFGF and EGF solutions were each added to DMEM/F12 (DMEM/F-12, HEPES) to a final concentration of 40 ng/mL and 20 ng/mL, respectively, to prepare the serum-free medium.

Serum was added to the serum-free medium to a final concentration of 10%, resulting in serum-containing medium (PS-). Penicillin-Streptomycin solution was added to the serum-containing medium (PS-) to a concentration of 50 units/mL and 50 µg/mL, respectively, resulting in serum-containing medium (PS+).

### <Isolation of Cardiac Stem Cells from Human Pediatric Heart Tissue>

### (Procedure 1: Isolation of Human Pediatric Cardiac Stem Cells)

The heart tissue collected from a human pediatric subject was washed in DPBS (-) and then minced while moistening with DPBS (-). DNase I was dissolved in injectable water, and a DNase I solution was prepared. Collagenase was dissolved in injectable water, and a collagenase solution was prepared using DPBS (+). The DNase I solution was added to the collagenase solution, creating a tissue dissociation reagent. The tissue dissociation reagent was dropped onto the minced tissue. The tissue dissociation reagent containing cells was collected in a centrifuge tube and incubated at 37°C. The cell solution was filtered using a cell strainer, and the filtrate was collected in a centrifuge tube. The collected cell solution was centrifuged, and the supernatant was discarded. Add 1 mL of serum medium (PS+) warmed to 37°C and resuspended the cell pellet by pipetting. The cell suspension was seeded into a flask and incubated in a CO₂ incubator (37°C, 5% CO₂).

### (Procedure 2: Primary Culture)

The supernatant containing cells from the flask incubated in Procedure 1 was collected and cultured in a CO₂ incubator (37°C, 5% CO₂). The medium was replaced with serum-containing medium (PS-) at appropriate intervals, and the cells were cultured for 3 to 14 days.

### (Procedure 3: Expansion Culture)

The culture supernatant from the flask after primary culture was removed, and the culture surface was washed with DPBS (-). 1×Trypsin/EDTA solution was added and mixed evenly, followed by incubation in a CO₂ incubator (37°C, 5% CO₂). The cells were collected with serum-containing medium (PS-) and expansion culture was started in the CO₂ incubator (37°C, 5% CO₂). The serum-containing medium (PS-) was replaced at appropriate intervals.

### (Procedure 4: Cryopreservation)

The culture supernatant from the flask after expansion culture was removed, and the culture surface was washed with DPBS (-). 1×Trypsin/EDTA was added and incubated. After confirming cell detachment, serum-free medium was added, and the cell suspension was collected into a 50 mL centrifuge tube and centrifuged. After centrifugation, the supernatant was discarded, and serum-containing medium (PS-) was added to resuspend the pellet. The number of cells in the cell suspension was measured, and an equal amount of cryoprotectant solution (CP-1) was added to the appropriately diluted cell suspension in serum medium (PS-), which was then stored frozen.

### <Preparation Method of Human Pediatric-derived Cardiac Stem Cells (Lot: FBS-pCSC#1, FBS-pCSC#2)>

Human pediatric-derived cardiac stem cells were cultured and cryopreserved from human heart tissue following the method described in Procedures 1-4 (Passage No. 2). Bovine fetal serum (BIOSERA, FB-1380 or HyClone, SH30084.03) was used as serum.

The cryopreserved cells were thawed and cultured using bovine fetal serum, and human pediatric-derived cardiac stem cells prepared by subculturing were used as test cells for the experiment.

### <Preparation Method of Control Human Pediatric-derived Cardiac Stem Cells (Lot: HS-pCSC#1)>

Human pediatric-derived cardiac stem cells were manufactured and cryopreserved from human pediatric heart tissue following the method described in Procedures 1-4 (Passage No. 2). Adult pool serum (Sigma, T6914) was used as serum.

The cryopreserved cells were thawed and cultured using bovine fetal serum, and human pediatric-derived cardiac stem cells prepared by subculturing were used as test cells for the experiment.

### <Preparation Method of Human Adult-derived Cardiac Stem Cells (Lot: aCSC#1)>

Human adult cardiac tissue collected from a human adult was used to isolate cardiac stem cells following the same method described in "Isolation of Cardiac Stem Cells from Human Pediatric Heart Tissue" (Procedure 1-4). The isolated cells were cryopreserved and subcultured to prepare human adult-derived cardiac stem cells for use as test cells in the experiment. Bovine fetal serum was used as serum.

### [Example 2: Analysis of Cell Surface Antigens and Intracellular Antigens Expression]

Based on the cell count measurement results, the test cells were prepared to a concentration of 1×10⁶ cells/mL, and the expression of cell surface antigens and intracellular antigens were measured using flow cytometry according to a method known to those skilled in the art. The results are shown in Tables 2 and 3.

The antibodies used are shown in Table 1.

**[Table 1]**

| Table 1. List of antibodies used | | | |
|---|---|---|---|
| Designation | Product name | Manufacturer | Product number |
| CD105 | APC Mouse Anti-Human CD105 | BD | 562408 |
| CD90 | APC anti-Human CD90(Thy1) | Biolegend Inc. | 328114 |
| SIRPA | APC anti-Human CD172a/b(SIRP *α* / *β*) | Biolegend Inc. | 323809 |
| CD34 | PE Mouse Anti-Human CD34 | BD | 348057 |
| CD31 | PE Mouse Anti-Human CD31 | BD | 340297 |
| CD29 | PE Mouse Anti-Human CD29 | BD | 555443 |
| c-kit | PE Mouse Anti-Human CD117 | BD | 340529 |
| DDR2 | Anti-DDR2 antibody | Abcam plc. | ab63337 |
| | Goat Anti-Mouse IgG H&L (Alexa Fluor^{®}488) | Abcam plc. | ab150113 |
| Tropomyosin | Monoclonal Anti-Tropomyosin (Sarcomeric) | Sigma-Aldrich | T9283-.2ML |
| | PE Rat Anti-Mouse IgG₁ | BD | 340270 |
| CD45 | FITC Mouse Anti-IIuman CD45 | BD | 347463 |

As a result, human pediatric-derived cardiac stem cells showed consistently high percentages of CD105-positive cells, while the percentages of CD45 and c-kit-positive cells were consistently low. In particular, the percentage of c-kit-positive cells was found to be lower compared to the control cells (lot: HS-pCSC#1). Additionally, both FBS-pCSC#1 and FBS-pCSC#2 demonstrated high percentages of SIRPA and CD29-positive cells.

**[Table 2]**

| Table 2. Measurement Results List | | | | |
|---|---|---|---|---|
| Lot | | FBS-pCSC#1 | FBS-pCSC#2 | HS-pCSC#1 |
| Positive cell rate of cell surface antigens (%) | CD105 | 98 | 99 | 99 |
| | CD90 | 90 | 84 | 38 |
| | SIRPA | 84 | 79 | 88 |
| | CD45 | 1 | 0 | 0 |
| | CD34 | 20 | 14 | 14 |
| | CD31 | 23 | 29 | 83 |
| | c-kit | 8 | 3 | 22 |
| | CD29 | 99 | 99 | 99 |
| Positive cell rate of intracellular antigens (%) | DDR2 | 94 | 28 | 92 |
| | Tropomyosin | 11 | 16 | 13 |

On the other hand, human adult-derived cardiac stem cells exhibited a low percentage of CD90-positive cells and a high percentage of CD31-positive cells, suggesting that they may be different from human pediatric-derived cardiac stem cells.

**[Table 3]**

| Table 3. Measurement Results List | | |
|---|---|---|
| Lot | | aCSC#1 |
| Positive cell rate of cell surface antigens (%) | CD105 | 99 |
| | CD90 | 1 |
| | SIRPA | 59 |
| | CD45 | 0 |
| | CD34 | 1 |
| | CD31 | 99 |
| | c-kit | 10 |
| | CD29 | 99 |

### [Example 3: Analysis of Gene Expression]

Gene expression was analyzed using real-time PCR, following methods known to those skilled in the art, with the prepared test cells. The results are shown in Table 5.

**[Table 4]**

| Table 4. List of primers used | |
|---|---|
| Designation | Product name |
| GATA4 | TaqMan Gene Expression Assay |
| | Gene name: GATA4 Hs00171403 ml |
| Mef2C | TaqMan Gene Expression Assay |
| | Gene name: Mef2C Hs00231149 ml |
| Tbx5 | TaqMan Gene Expression Assay |
| | Gene name: Tbx5 Hs00361155 ml |
| Nkx2.5 | TaqMan Gene Expression Assay |
| | Gene name: Nkx2.5 Hs00231763 ml |
| MYH6 | TaqMan Gene Expression Assay |
| | Gene name: MYH6 Hs00411908_ml |
| MYL2 | TaqMan Gene Expression Assay |
| | Gene name: MYL2 Hs00166405_ml |
| ELN | TaqMan Gene Expression Assay |
| | Gene name: ELN Hs00355783_ml |
| TNNT2 | TaqMan Gene Expression Assay |
| | Gene name: TNNT2 Hs00165960_ml |
| *β* -actin | TaqMan Gene Expression Assay ACTB |
| | Gene name: Hs99999903_ml |

| | |
|---|---|
| (All manufacturers are Themo Fisher Scientific Inc., and the product number is 4331182.) | |

The relative expression levels of genes in a cell population composed of human adult heart-derived fibroblasts (LONZA, NHCF-A, CC-2903) were measured, with the expression levels normalized to beta-actin and the baseline set to 1.0. The relative expression levels of each gene in the various groups were then compared. As a result, among the genes known to be involved in cardiomyocyte differentiation (Nkx2.5, Gata4, Tbx5, Mef2C), the relative expression levels of Gata4 and Tbx5 in FBS-pCSC#1 and FBS-pCSC#2 were found to exceed 1.0, respectively (Table 5). Additionally, the expression level of MYH6 (αMHC), a known cardiomyocyte marker, was below the detection sensitivity (indicated as N/A in the table). Furthermore, the expression of Elastin (ELN), a known fibroblast marker, was 0.10 times (FBS-pCSC#1) and 0.18 times (FBS-pCSC#2) that of the baseline, respectively.

**[Table 5]**

| Table 5. Measurement results List | | | | | |
|---|---|---|---|---|---|
| Lot | | | FBS-pCSC#1 | FBS-pCSC#2 | HS-pCSC#1 |
| Gene expression | Cardiac differentiation transcription factor | Nkx2.5 | N/A | 0.42 | N/A |
| | | Gata4 | 1.93 | 2.10 | 0.75 |
| | | Tbx5 | 4.11 | 3.59 | 0.34 |
| | | Mef2C | 3.70 | 3.97 | 3.04 |
| | | MYL2 | 2.79 | 2.69 | 4.32 |
| | | MYH6 | N/A | N/A | N/A |
| | | ELN | 0.10 | 0.18 | 0.01 |
| | | TNNT2 | 1.32 | 0.75 | 0.08 |

### [Example 4: Measurement of HGF Production]

### <Preparation of Culture Supernatant of Human-derived cardiac stem cells>

The test cells (human-derived cardiac stem cells) were seeded in a 24-well plate (medium volume 0.5 mL/well) and cultured for 3 days in a CO₂ incubator (5% CO₂, 37°C), after which the supernatant was collected.
- Seeding cell densities: 0.63×10⁴ cells/cm², 1.00×10⁴ cells/cm², 1.25×10⁴ cells/cm², 2.00×10⁴ cells/cm², 2.50×10⁴ cells/cm², 3.35×10⁴ cells/cm²

### <Measurement of HGF Production>

The prepared culture supernatant was used to measure the HGF levels using an ELISA kit (HGF, Human, ELISA Kit, Quantikine, R&D Systems, DHG00B). The ELISA was performed following the manufacturer's instructions, and absorbance was measured using a plate reader (Bio-Rad Laboratories, iMARK). The data was then analyzed using Microplate Manager 6 (Bio-Rad).

The quantitative results are shown in Table 6.

As a result, in all seeding cell densities, cardiac stem cells cultured with FBS (FBS-pCSC#1, FBS-pCSC#2) showed a tendency to produce higher amounts of HGF compared to the cardiac stem cells cultured with adult pooled serum (HS-pCSC#1). Additionally, the HGF production from adult-derived cardiac stem cells (aCSC#1) was below the quantification detection limit.

**[Table 6]**

| Table 6. Quantitative Results of HGF | | |
|---|---|---|
| Lot | Seeding cell number (cells/cm²) | HGF production (pg/mL) |
| FBS-pCSC#1 | 0.63 × 10⁴ | 8,960 |
| | 1.00 × 10⁴ | 14,361 |
| | 1.25 × 10⁴ | 17,542 |
| | 2.00 × 10⁴ | 31,864 |
| | 2.50 × 10⁴ | 27,109 |
| | 3.35 × 10⁴ | 47,993 |
| FBS-pCSC#2 | 0.63 × 10⁴ | 28,073 |
| | 1.00 × 10⁴ | 53,123 |
| | 1.25 × 10⁴ | 54,492 |
| | 2.00 × 10⁴ | 85,019 |
| | 2.50 × 10⁴ | 126,718 |
| | 3.35 × 10⁴ | 142,226 |
| HS-pCSC#1 | 0.63 × 10⁴ | 697 |
| | 1.00 × 10⁴ | 905 |
| | 1.25 × 10⁴ | 821 |
| | 2.00 × 10⁴ | 1,237 |
| | 2.50 × 10⁴ | 1,283 |
| | 3.35 × 10⁴ | 2,093 |
| aCSC#1 | 2.00 × 10⁴ | <300 |

### [Example 5: Co-culture with Human PBMC and Measurement of IFN-γ Production]

### <Seeding and Pre-culture of Human-derived cardiac stem cells>

The test cells (human-derived cardiac stem cells) were seeded into a 24-well plate (1 mL medium/well) and cultured overnight in a CO₂ incubator (5% CO₂, 37°C).
- Seeding cell densities: 0.63×10⁴ cells/cm², 1.25×10⁴ cells/cm², 2.50×10⁴ cells/cm², 3.75×10⁴ cells/cm²

### <Co-culture of Human-derived cardiac stem cells with Human PBMC>

PBMC (iQ Biosciences, IQB-PBMC102) were thawed in a 37°C water bath, and live cell counts were determined using a cell counter. Based on the live cell count, PBMC culture medium (without P/S) was added to adjust the cell concentration to 5×10⁵ cells/900 µL. The prepared PBMC were added to the wells containing human-derived cardiac stem cells prepared as described in "Seeding and Pre-culture of Human-derived cardiac stem cells" at an amount of 900 µL/well.

For the wells with PHA stimulation, PHA (Sigma-Aldrich, L1668) was prepared to a final concentration of 5 µg/mL using PBMC culture medium (without P/S) at 50 µg/mL, and 100 µL/well was added to each well. For the wells without PHA stimulation, 100 µL/well of PBMC culture medium (without P/S) was added.

The co-cultures were incubated for 3 days in a CO₂ incubator (5% CO₂, 37°C), after which the supernatants were collected.

### <Measurement of IFN-γ Production>

The prepared culture supernatants were used to measure IFN-γ levels using an ELISA kit (abcam, ab46025). The ELISA was performed according to the manufacturer's instructions, and absorbance was measured using a plate reader. The data were analyzed using Microplate Manager 6 (Bio-Rad).

### <Comparison of IFN-γ Production from Co-culture of Human-derived cardiac stem cells and Human PBMC>

The quantitative results of the measured IFN-γ production are shown in Table 7-1 to Table 7-3, and the relative production is shown in Figure 1.

In the case of PHA stimulation, human-derived cardiac stem cells alone did not produce IFN-γ (Table 7-2). However, when PHA was applied, PBMC produced IFN-γ (Table 7-1). This suggests that PHA stimulation activated T-cells, leading to the production of IFN-γ. Therefore, it was demonstrated that immune modulation and anti-inflammatory effects can be evaluated in this experimental system.

In the case without PHA stimulation, no significant IFN-γ production was observed in the co-culture of PBMC and human-derived cardiac stem cells (Table 7-3). This suggests that human-derived cardiac stem cells may have low immunogenicity.

Furthermore, in the case of PHA stimulation, the IFN-γ production in the co-culture of PBMC and human-derived cardiac stem cells decreased in a seeding cell number-dependent manner (Table 7-1). This suggests that human-derived cardiac stem cells may possess immune-modulatory and/or anti-inflammatory properties. Additionally, when the seeding cell number of human-derived cardiac stem cells was 1/20 or greater compared to the PBMC seeding cell number, IFN-γ production was suppressed by more than 90% in all three cases of human-derived cardiac stem cells. This suggests that human-derived cardiac stem cells have relatively strong immune-modulatory and/or anti-inflammatory effects. Moreover, it was shown that human-derived cardiac stem cells cultured with FBS (FBS-pCSC#1, FBS-pCSC#2) produced lower relative amounts of IFN-γ compared to human-derived cardiac stem cells cultured with adult pooled serum (HS-pCSC#1). This suggests that human-derived cardiac stem cells cultured with FBS (FBS-pCSC#1, FBS-pCSC#2) have a higher potential for suppressing IFN-γ production.

**[Table 7-1]**

| Table 7-1. IFN-gamma production volume (1/3) | | | | | | | |
|---|---|---|---|---|---|---|---|
| PBMC | PHA stimulation | Cardiac stem cell | CSC Lot # | Number of seeded cells of CSCs (cells/cm²) | The ratio of seeded cell number of CSCs to the seeded cell number of PBMCs | IFN-gamma production volume (pg/mL) | Relative production volume of IFN-gamma * (pg/mL) |
| + | + | - | - | - | - | 77,641 | 1.00 |
| + | + | + | FBS-pCSC#1 | 0.63 × 10⁴ | 1/40 | 6,110 | 0.08 |
| | | | | 1.25 × 10⁴ | 1/20 | 2,803 | 0.04 |
| | | | | 2.50 × 10⁴ | 1/10 | 1,992 | 0.03 |
| | | | | 3.75 × 10⁴ | 1/6.7 | 1,721 | 0.02 |
| | | | FBS-pCSC#2 | 0.63 × 10⁴ | 1/40 | 8,186 | 0.11 |
| | | | | 1.25 × 10⁴ | 1/20 | 3,962 | 0.05 |
| | | | | 2.50 × 10⁴ | 1/10 | 2,259 | 0.03 |
| | | | | 3.75 × 10⁴ | 1/6.7 | 1,584 | 0.02 |
| | | | HS-pCSC#1 | 0.63 × 10⁴ | 1/40 | 23,724 | 0.31 |
| | | | | 1.25 × 10⁴ | 1/20 | 7,422 | 0.10 |
| | | | | 2.50 × 10⁴ | 1/10 | 5,464 | 0.07 |
| | | | | 3.75 × 10⁴ | 1/6.7 | 4,492 | 0.06 |

| | | | | | | | |
|---|---|---|---|---|---|---|---|
| * Relative production volume of IFN-gamma when the amount produced in the case of PBMC culture alone (with PHA stimulation) is set to 1. | | | | | | | |

**[Table 7-2]**

| Table 7-2. IFN-gamma production volume (2/3) | | | | | |
|---|---|---|---|---|---|
| PBMC | PHA stimulation | Cardiac stem cell | CSC Lot # | Number of seeded cells of CSCs (cells/cm²) | IFN-gamma production volume (pg/mL) |
| - | + | + | FBS-pCSC#1 | 0.63 × 10⁴ | N.D.^{*1} |
| | | | | 1.25 × 10⁴ | N.D.^{*1} |
| | | | | 2.50 × 10⁴ | N.D.^{*1} |
| | | | | 3.75 × 10⁴ | N.D.^{*1} |
| | | | FBS-pCSC#2 | 0.63 × 10⁴ | N.D.^{*1} |
| | | | | 1.25 × 10⁴ | N.D.^{*1} |
| | | | | 2.50 × 10⁴ | N.D.^{*1} |
| | | | | 3.75 × 10⁴ | N.D.^{*1} |
| | | | HS-pCSC#1 | 0.63 × 10⁴ | N.D.^{*1} |
| | | | | 1.25 × 10⁴ | N.D.^{*1} |
| | | | | 2.50 × 10⁴ | N.D.^{*1} |
| | | | | 3.75 × 10⁴ | N.D.^{*1} |

| | | | | | |
|---|---|---|---|---|---|
| *1 N.D.: Below the detection limit (detection limit: 5 pg/mL) | | | | | |

**[Table 7-3]**

| Table 7-3. IFN-gamma production volume (3/3) | | | | | | |
|---|---|---|---|---|---|---|
| PBMC | PHA stimulation | Cardiac stem cell | CSC Lot # | Number of seeded cells of CSCs (cells/cm²) | The ratio of seeded cell number of CSCs to the seeded cell number of PBMCs | IFN-gamma production volume (pg/mL) |
| + | - | - | - | - | - | N.D.^{*1} |
| + | - | + | FBS-pCSC#1 | 0.63 × 10⁴ | 1/40 | N.D.^{*1} |
| | | | | 1.25 × 10⁴ | 1/20 | N.D.^{*1} |
| | | | | 2.50 × 10⁴ | 1/10 | N.D.^{*1} |
| | | | | 3.75 × 10⁴ | 1/6.7 | <12.5^{*2} |
| | | | FBS-pCSC#2 | 0.63 × 10⁴ | 1/40 | N.D.^{*1} |
| | | | | 1.25 × 10⁴ | 1/20 | N.D.^{*1} |
| | | | | 2.50 × 10⁴ | 1/10 | N.D.^{*1} |
| | | | | 3.75 × 10⁴ | 1/6.7 | <12.5^{*2} |
| | | | HS-pCSC#1 | 0.63 × 10⁴ | 1/40 | N.D.^{*1} |
| | | | | 1.25 × 10⁴ | 1/20 | N.D.^{*1} |
| | | | | 2.50 × 10⁴ | 1/10 | N.D.^{*1} |
| | | | | 3.75 × 10⁴ | 1/6.7 | <12.5^{*2} |

| | | | | | | |
|---|---|---|---|---|---|---|
| *1 N.D.: Below the detection limit (detection limit: 5 pg/mL) *2 Below the quantification limit (Quantification limit: 12.5 pg/mL) | | | | | | |

### [Example 6: Evaluation of Differentiation Characteristics into Cardiomyocytes]

As part of the characterization of human-derived cardiac stem cells (hCSCs), their differentiation potential into cardiomyocytes was evaluated. Specifically, human-derived cardiac stem cells were co-cultured with mouse cardiomyocytes, and the expression of cardiomyocyte-related markers, morphological characteristics, and functional properties were examined.

### <Preparation, Seeding, and Culture of Mouse Cardiomyocytes>

Pregnant 14-day-old BALB/cCrSlc mice were euthanized, and hearts were excised from embryos at 14 days of gestation. Using small scissors, a portion of the excised heart was cut, washed internally, and then treated with 0.05% trypsin/EDTA solution at 37°C with shaking for 10 minutes. After confirming the turbidity of the trypsin/EDTA solution, the cell suspension was collected, and the same procedure was repeated to collect additional cell suspensions. The collected cell suspensions were centrifuged, the supernatant was discarded, and the cell pellet was resuspended in mouse cardiomyocyte culture medium. The cell suspension was filtered using a 100 µm cell strainer and seeded into a glass petri dish (Duran, 217554804). After incubating in a CO₂ incubator (37°C, 5% CO₂) for 60 minutes, the culture medium containing non-adherent cells was collected into a 50 mL tube, and fibroblasts were removed.

The prepared mouse cardiomyocytes were seeded into each well of a gelatin-coated chamber cover (8-well) at a cell density of 1.0×10⁵ cells/cm² (medium volume: 0.3 mL/well). For wells used for the culture of human-derived cardiac stem cells alone, the same amount of medium was added. The cells were cultured in a CO₂ incubator (37°C, 5% CO₂) for approximately 24 hours.

### [Induction of Differentiation of Human-derived cardiac stem cells into Cardiomyocytes]

1. 5-Azacytidine Treatment (5-AzaC)
   The medium from the thawed human-derived cardiac stem cells (10% FBS medium) was completely aspirated and replaced with medium containing 10 µM 5-AzaC and 10% FBS (10 mL/75 cm² flask). The cells were cultured in a CO₂ incubator (37°C, 5% CO₂) for approximately 24 hours.
2. Preparation of Differentiation-Induced Human-derived cardiac stem cells Cells in the 75 cm² flask were harvested, and a portion of the cell suspension was sampled and counted for live cells using a cell counter. The entire cell suspension was centrifuged, and the supernatant was discarded. Differentiation induction medium was added, and the cell suspension for differentiation-induced human-derived cardiac stem cells was prepared. The differentiation induction medium (containing phenol red) was prepared by mixing 10% FBS medium and mouse cardiomyocyte medium (containing phenol red) at a 1:1 ratio.
3. Co-culture with Mouse Cardiomyocytes
   - To each well of the 24-hour cultured mouse cardiomyocytes, an equal volume (0.3 mL/well) of differentiation-induced human cardiac stem cell suspension (5×10⁴ cells/cm²) was added (total medium volume: 0.6 mL/well).
   - For the culture of human-derived cardiac stem cells alone, the differentiation-induced human cardiac stem cell suspension (5×10⁴ cells/cm²) was added to wells without seeded mouse cardiomyocytes.
   - Co-culture and single culture were initiated in a CO₂ incubator (37°C, 5% CO₂).
   - After 3 and 5 days of co-culture, the beating activity of the cells was observed under phase-contrast or fluorescence microscopy, and the expression of cardiomyocyte-related markers and morphological characteristics were analyzed.

### [Confirmation of Cardiomyocyte-Related Markers by Immunostaining]

The medium was removed from the chamber covers containing the cultured cells, and the cells were fixed in 4% PFA solution at room temperature for 15 minutes. The cells were then permeabilized using 0.1% Triton-X solution. Primary antibody staining was performed for 1 hour at room temperature using the antibodies listed in Table 8. Secondary antibody staining was performed for 30 minutes (protected from light) at room temperature using the antibodies listed in Table 9. Blocking solution was prepared using PBS-T solution containing 1% BSA.

**[Table 8]**

| Table 8. Primary antibody solution used | | |
|---|---|---|
| Antibody solution name | Antibody reagent name | Dilution rate |
| Antibody solution① | Anti-Mouse/Human cTnT antibody (Anti-Cardiac Troponin T antibody [1C11], Abcam plc., ab8295) | 1/300 |
| | Anti- Human Lamin antibody (Anti-Lamin A+C antibody [EPR4100], Abcam plc., ab108595) | 1/250 |
| Antibody solution② | Anti-Mouse/Human Actinin antibody (Monoclonal Anti- *α* -Actinin(Sarcomeric) antibody produced in mouse, Sigma-Aldrich, A7811) | 1/300 |
| | Anti- Human Lamin antibody (Anti-Lamin A+C antibody [EPR4100], Abcam plc., ab108595) | 1/250 |

**[Table 9]**

| Table 9. Secondary antibody solution used | |
|---|---|
| Antibody reagent name | Dilution rate |
| Alexa Fluor 488 labeled-anti-Rabbit IgG antibody (Goat anti-Rabbit IgG (H+L) Superclonal Recombinant Secondary Antibody, Alexa Fluor 488, Thermo Fisher Scientific Inc., A27034) | 1/500 |
| Alexa Fluor 555 labeled-anti-Mouse IgG antibody (Goat anti-Mouse IgG (H+L) Superclonal Recombinant Secondary Antibody, Alexa Fluor 555, Thermo Fisher Scientific Inc., A28180) | 1/500 |

Additionally, each staining was performed using DAPI solution.

For the immunostained wells, an appropriate amount of fluorescent quenching agent-containing mounting medium was added. The antibody staining results were observed under a fluorescence microscope (Keyence Corporation, BZ-X710) using a 20x objective lens.

### [Overview of Differentiation Characteristics Evaluation of Human-derived cardiac stem cells]

An overview of the differentiation characteristics evaluation results of human-derived cardiac stem cells (FBS-PCS#1 and FBS-PCS#2) is shown in Table 10. In the table, the expression of cardiomyocyte-related markers is denoted as follows:
- "+" indicates that Lamin-positive and cTnT-positive cells or Lamin-positive and Actinin-positive cells were observed in the field of view.
- "-" indicates that neither Lamin-positive and cTnT-positive cells nor Lamin-positive and Actinin-positive cells were observed in the field of view.

Regarding morphological characteristics:
- "+" indicates the formation of sarcomeres was confirmed.
- "-" indicates that sarcomere formation was not observed.

Regarding functional characteristics:
- "+" indicates that beating cells were observed.
- "-" indicates that beating cells were not observed.

**[Table 10]**

| Table 10. Overview of the results of differentiation characteristic evaluation | | | |
|---|---|---|---|
| | | Lot | |
| | | FBS-pCSC#1 | FBS-pCSC#2 |
| Expression of cardiomyocyte-related markers | | | |
| cTnT | 3 days after the start of co-culture | + | + |
| | 5 days after the start of co-culture | + | + |
| | 3 days after the start of cultured alone | - | - |
| | 5 days after the start of cultured alone | - | - |
| Actinin | 3 days after the start of co-culture | + | + |
| | 5 days after the start of co-culture | + | + |
| | 3 days after the start of cultured alone | - | - |
| | 5 days after the start of cultured alone | - | - |

| Morphological characteristics (Sarcomere formation) | | | |
|---|---|---|---|
| 3 days after the start of co-culture | | + | + |
| 5 days after the start of co-culture | | + | + |
| 3 days after the start of cultured alone | | - | - |
| 5 days after the start of cultured alone | | - | - |

| Function (pulsation) | | | |
|---|---|---|---|
| 3 days after the start of co-culture | | + | + |
| 5 days after the start of co-culture | | + | + |
| 3 days after the start of cultured alone | | - | - |
| 5 days after the start of cultured alone | | - | - |

### [Expression of Cardiomyocyte-Related Markers]

Human-derived cardiac stem cells (the test cells) were co-cultured with mouse cardiomyocytes, and immunostaining was performed 3 and 5 days after the start of co-culture. The expression of Lamin, a human cell-specific antigen, as well as cardiomyocyte-related markers such as Cardiac Troponin T (hereafter, cTnT) and α-Actinin (hereafter, Actinin) was confirmed.

The immunostaining images from this experiment are shown in Figures 2 and 3. Figure 2 shows the expression of the cardiomyocyte-related marker (cTnT) 5 days after the start of co-culture of mouse cardiomyocytes and human-derived cardiac stem cells, and Figure 3 shows the expression of the cardiomyocyte-related marker (Actinin) 5 days after the start of co-culture.

Additionally, immunostaining was performed on the human-derived cardiac stem cells alone. The results showed that in the case of human-derived cardiac stem cells cultured alone, the expression of Lamin was confirmed, but the expression of cardiomyocyte-related markers was not observed.

### [Morphological Characteristics]

As shown in Figures 2 and 3, in the co-culture of mouse cardiomyocytes and human-derived cardiac stem cells, both FBS-PCS#1 and FBS-PCS#2 human-derived cardiac stem cells exhibited sarcomere (striated structure) formation at 3 and 5 days after the start of co-culture. In contrast, sarcomere formation was not observed in the human-derived cardiac stem cells cultured alone.

### [Functional Characteristics]

In the co-culture of mouse cardiomyocytes and human-derived cardiac stem cells, both FBS-PCS#1 and FBS-PCS#2 human-derived cardiac stem cells exhibited beating activity 3 and 5 days after the start of co-culture.

Based on the above, both FBS-PCS#1 and FBS-PCS#2 human-derived cardiac stem cells demonstrated differentiation characteristics into cardiomyocytes. In the co-culture with mouse cardiomyocytes, expression of cardiomyocyte-related markers such as cTnT and Actinin was confirmed. Additionally, morphological characteristics such as the formation of sarcomeres and functional characteristics such as the presence of beating activity were also observed.

### [Example 7: Measurement of ADM Production]

### [Preparation of Human Stem Cell Culture Supernatant]

As test cells, human pediatric-derived cardiac stem cells (pCSC) and human adult-derived cardiac stem cells (aCSC) prepared according to the method described in Example 1, as well as human adult bone marrow-derived mesenchymal stem cells (hMSC) (Lonza, PT-2501), were used. The hMSCs, which are known as representative somatic stem cells, were employed as a control in this example. Two conditions were prepared for each cell type: cells stimulated with cytokines (TNF-α and IL-4) for three days, and unstimulated cells. The cells were seeded into T-25 flasks at a density of 5.0 × 10⁵ cells per flask (2.0 × 10⁴ cells/cm²) and pre-cultured in a CO₂ incubator (5% CO₂, 37°C). On the following day, the culture medium was completely removed, and 5 mL of DMEM (High Glucose; Wako, 043-30085) supplemented with 1% penicillin-streptomycin (Wako, 168-23191) was added. After five days of culture, the conditioned medium (supernatant) was collected for analysis.

### [Measurement of ADM Production]

The amount of adrenomedullin (ADM) produced in the collected conditioned medium was quantified using an ELISA kit (Human ADM (Adrenomedullin) ELISA Kit, Elabscience^{®}, E-EL-H0275). The measurement was performed in accordance with the manufacturer's instructions provided with the kit. The quantitative results are shown in Table 11. Both pCSC and aCSC exhibited higher levels of ADM production compared to the control hMSC. In all cell types, cytokine stimulation resulted in increased ADM secretion.

### [Table 11]

**Table 11**

| sample No. | human ADM concentration (pg/mL) | |
|---|---|---|
| | unstimulated cells (-) | stimulated cells with cytokines (+) |
| pCSC-01 | 48,979 | >250,000 |
| pCSC-02 | 114,802 | >250,000 |
| pCSC-03 | 50,563 | >250,000 |
| aCSC | 82,988 | >250,000 |
| hMSC-01 | 1,272 | 3,761 |
| hMSC-02 | 1,284 | 7,331 |
| hMSC-03 | 1,213 | 15,837 |

### [Example 8: Measurement of MMP1 Production]

### [Preparation of Conditioned Medium from Human Stem Cells]

Conditioned media from human stem cells (pCSC, aCSC, and hMSC) were prepared using the same method as described in Example 7.

### [Measurement of MMP1 Production]

The collected conditioned media were analyzed to quantify MMP1 production using a multiplex assay kit (ProcartaPlex Multiplex Immunoassays, Thermo Fisher, PPX-23-MXT2A7T). The measurement was performed in accordance with the manufacturer's instructions provided with the kit. The quantitative results are shown in Table 12. Both pCSC and aCSC exhibited higher levels of MMP1 production compared to the control hMSC. In addition, cytokine stimulation resulted in increased MMP1 secretion in hMSCs.

### [Table 12]

**Table 12**

| sample No. | human MMP1 concentration (pg/mL) | |
|---|---|---|
| | unstimulated cells (-) | stimulated cells with cytokines (+) |
| pCSC-01 | >28,200 | |
| pCSC-02 | >28,200 | |
| pCSC-03 | >28,200 | |
| aCSC | >28,200 | |
| hMSC-01 | 67 | 165 |
| hMSC-02 | 111 | 693 |
| hMSC-03 | 303 | 2,518 |

### [Example 9: Evaluation Using a Myocardial Infarction (Complete Occlusion of the Coronary Artery) Model]

A myocardial infarction model in nude rats (CLEA Japan, Inc., F344/NJcl-rnu/rnu, male, 9 weeks old) was used to investigate the myocardial functional improvement effects following intramyocardial administration of the test substance.

### [Creation of Myocardial Infarction (Complete Occlusion of the Coronary Artery) Model]

Following subcutaneous administration of the analgesic meloxicam(MELOXIRIN^{®} INJ., Fujita Pharmaceutical Co., Ltd.), rats were anesthetized via inhalation of 0.5-2.0% isoflurane (Isoflurane Inhalation Solution "VTRS", Mylan EPD G.K.) using a small animal anesthetic device. Under mechanical ventilation, an appropriate dose of lidocaine (Lidocaine 2% Syringe for Intravenous Injection "Terumo", Terumo Corporation) was administered subcutaneously. A thoracotomy was then performed at the lateral chest wall to expose the heart. The left anterior descending (LAD) coronary artery was completely ligated using a suture with a needle (ELP Suture with Needle M10-60B2, Akiyama Seisakusho Co., Ltd.).

During the procedure, electrocardiograms (lead II) were recorded via LabChart Pro (AD Instruments) to monitor ST-segment elevation, and myocardial pallor was visually observed to confirm successful occlusion. The chest was then closed, and the incision was sutured. The day of myocardial infarction model creation was designated as Day 0, and the following day as Day 1.

### [Preparation of Test Substance]

A cell suspension of human-derived cardiac stem cells (CSCs) was used as the test substance. The CSCs used in this example were derived from pediatric tissue in this example. The cell suspension was prepared to achieve a cell density of 1×10⁵ cells/50 µL or 2×10⁵ cells/50 µL. As a control substance, STEM-CELLBANKER GMP grade (Nippon Zenyaku Kogyo Co., Ltd., CB045) was used.

### [Administration of Test Substance to Myocardial Infarction (Complete Occlusion of the Coronary Artery) Model]

Within 15 to 22 minutes after creating the myocardial infarction model, the test substance or control substance was administered intramyocardially into the myocardial tissue near the infarct site of the left ventricle. Intramyocardial injection was performed using an insulin syringe (NIPRO Myshot 30G, Nipro Corporation). 50 µL of the test substance or control substance was divided into three equal parts and injected intramyocardially at three sites near the infarct region of the left ventricle (referred to as the CSC group or Control group). The observation period was 8 weeks following the creation of the myocardial infarction model.

### [Inclusion Criteria for Myocardial Infarction Model]

In previous studies using the same rat strain, body weight loss observed one week after myocardial infarction model induction was consistently within the range of 2-8% compared to preoperative values. Accordingly, animals exhibiting a body weight loss of ≥10% from baseline were considered to have undergone excessive surgical stress, which may have affected cardiac function. These animals were excluded from the present evaluation. As a result, the final analysis was conducted using data from 22 animals that met the inclusion criteria.

### [Body Weight Measurement]

Body weight was measured weekly using an electronic balance (UW4200S, Shimadzu Corporation) from before myocardial infarction model induction until 8 weeks after intramyocardial administration (at the time of necropsy) of the test or control substance. No significant differences in body weight were observed between the CSC-treated groups (1×10⁵ cells and 2×10⁵ cells) and the Control group throughout the observation period.

### [Echocardiographic Assessment]

Echocardiography was performed prior to myocardial infarction model induction and at 2, 4, 6, and 8 weeks after intramyocardial administration of the test or control substance. Inhalation anesthesia was induced using 2.0% isoflurane (Isoflurane Inhalation Solution "VTRS", Mylan EPD G.K.) delivered via a small-animal anesthesia system.

Cardiac ultrasound was conducted using a ultrasound imaging system (Vivid T9 Ultra Edition, GE Medical Systems). A linear probe (16 MHz) was placed on the rat's chest to obtain a parasternal short-axis view of the left ventricle. M-mode imaging was used to measure the left ventricular end-diastolic diameter (LVIDd), left ventricular end-systolic diameter (LVIDs), anterior wall thickness at end-diastole (LVAWd), posterior wall thickness at end-diastole (LVPWd), ejection fraction (EF), and fractional shortening (%FS). Each parameter was measured over three cardiac cycles, and the mean value was used for analysis.

The results of the echocardiography are shown in Table 13. "Pre" refers to the time point before the myocardial infarction model induction. Data are presented as mean ± S.E., with the numbers in parentheses indicating the sample size (n). Asterisks (*) denote a significant difference (P < 0.05), while parentheses around an asterisk (*) indicate a trend toward significance (P < 0.10) when compared to the Control group, as determined by Dunnett's test.

In the Control group, myocardial infarction resulted in impaired left ventricular (LV) systolic function, as evidenced by reductions in ejection fraction (EF) and fractional shortening (%FS), as well as cardiac remodeling characterized by increased LV internal diameters at end-diastole (LVIDd) and end-systole (LVIDs).

In the CSC-treated group receiving 1×10⁵ cells, a significant suppression of LVIDd enlargement was observed at 4 and 6 weeks post-injection compared to the Control group (P < 0.05), with a trend toward suppression at 8 weeks (P < 0.10). Significant suppression of LVIDs enlargement was also observed at 4, 6, and 8 weeks post-injection (P < 0.05). Additionally, a trend toward attenuation of the decrease in EF and %FS was noted at 2 weeks post-injection compared to the Control group (P < 0.10).

In the CSC-treated group receiving 2×10⁵ cells, a trend toward suppression of LVIDd enlargement was observed at 6 weeks post-injection (P < 0.10), while significant suppression of LVIDs enlargement was noted at 6 weeks (P < 0.05), with a trend toward suppression at 8 weeks (P < 0.10), compared to the Control group. Furthermore, at 2 weeks post-injection, a trend toward attenuation of EF and %FS reduction was also observed in this group (P < 0.10).

Based on these results, intramyocardial administration of CSCs in the myocardial infarction model demonstrated suppression of cardiac remodeling, as evidenced by inhibition of LVIDd and LVIDs enlargement, and a trend toward preservation of left ventricular systolic function, indicated by attenuation of EF and %FS decline. These findings suggest that CSC administration exerts cardioprotective effects, including inhibition of adverse remodeling and improvement of cardiac function.

**Table 13: Effects of CSC Administration on Echocardiographic Parameters in a Rat Model of Myocardial Infarction [Table 13-1]**

| **Group** | **Dose (cells 50 µL)** | **No. of animals** | **LVIDd (mm)** | | | | | **LVIDs (mm)** | | | | |
|---|---|---|---|---|---|---|---|---|---|---|---|---|
| | | | **Pre** | **2W** | **4W** | **6W** | **8W** | **Pre** | **2W** | **4W** | **6W** | **8W** |
| **Control** | **-** | **7** | **6.1 ±0.1** | **7.9 ±0.2 ⁽⁶⁾** | **9.0 ±0.1 ⁽⁶⁾** | **8.9 ±0.1 ⁽⁶⁾** | **92 ±0.2 ⁽⁶⁾** | **2.7 ±0.1** | **6.2 ±0.2 ⁽⁶⁾** | **7.2 ±0.1 ⁽⁶⁾** | **7.3 ±0.1 ⁽⁶⁾** | **7.6 ±0.1 ⁽⁶⁾** |
| **CSC** | **1×10⁵** | **7** | **6.4 ±0.1** | **7.7 ±0.2 ⁽⁶⁾** | **8.0 * ±0.3 ⁽⁶⁾** | **8.1 * ±0.1 ⁽⁶⁾** | **8.2 (*) ±0.3 ⁽⁶⁾** | **2.9 ±0.1** | **5.9 ±0.2 ⁽⁶⁾** | **6.3 * ±0.3 ⁽⁶⁾** | **6.6 * ±0.2 ⁽⁶⁾** | **6.7 * ±0.3 ⁽⁶⁾** |
| **CSC** | **2×10⁶** | **8** | **6.2 ±0.1** | **8.1 ±0.2 ⁽⁷⁾** | **8.9 ±0.3 ⁽⁷⁾** | **8.3 (*) ±0.2 ⁽⁷⁾** | **8.5 ±0.5 ⁽⁷⁾** | **2.8 ±0.1** | **6.2 ±0.1 ⁽⁷⁾** | **7.0 ±0.2 ⁽⁷⁾** | **6.6 * ±0.2 ⁽⁷⁾** | **7.0 (*) ±0.5 ⁽⁷⁾** |
| | | | | | | | | | | | | |

| **Group** | **Dose (cells 50 µL)** | **No. of animals** | **LVAWd (mm)** | | | | | **LVPWd (mm)** | | | | |
|---|---|---|---|---|---|---|---|---|---|---|---|---|
| | | | **Pre** | **2W** | **4W** | **6W** | **8W** | **Pre** | **2W** | **4W** | **6W** | **8W** |
| **Control** | **-** | **7** | **1.6 ±0.0** | **0.8 ±0.1⁽⁶⁾** | **0.7 ±0.0 ⁽⁶⁾** | **0.7 ±0.0 ⁽⁶⁾** | **0.7 ±0.0 ⁽⁶⁾** | **1.6 ±0.0** | **1.7 ±0.0 ⁽⁶⁾** | **1.7 ±0.0 ⁽⁶⁾** | **1.7 ±0.0 ⁽⁶⁾** | **1.7 ±0.0 ⁽⁶⁾** |
| **CSC** | **1×10⁵** | **7** | **1.6 ±0.0** | **0.9 ±0.1 ⁽⁶⁾** | **0.7 ±0.1 ⁽⁶⁾** | **0.7 ±0.0 ⁽⁶⁾** | **0.7 ±0.0 ⁽⁶⁾** | **1.6 ±0.0** | **1.7 ±0.0 ⁽⁶⁾** | **1.7 ±0.0 ⁽⁶⁾** | **1.7 ±0.0 ⁽⁶⁾** | **1.7 ±0.0⁽⁶⁾** |
| **CSC** | **2×10⁶** | **8** | **1.6 ±0.0** | **0.8 ±0.1 ⁽⁷⁾** | **0.8 ±0.1 ⁽⁷⁾** | **0.8 ±0.1 ⁽⁷⁾** | **0.7 ±0.1 ⁽⁷⁾** | **1.6 ±0.0** | **1.6 ±0.0 ⁽⁷⁾** | **1.6 ±0.0 ⁽⁷⁾** | **1.7 ±0.0 ⁽⁷⁾** | **1.7 ±0.0 ⁽⁷⁾** |

**[Table 13-2]**

| **Group** | **Dose (cells 50 µL)** | **No. of animals** | **EF (%)** | | | | | **%FS (%)** | | | | |
|---|---|---|---|---|---|---|---|---|---|---|---|---|
| | | | **Pre** | **2W** | **4W** | **6W** | **8W** | **Pre** | **2W** | **4W** | **6W** | **8W** |
| **Control** | **-** | **7** | **89.7 ±0.5** | **47.6 ±0.8 ⁽⁶⁾** | **45.6 ±1.2 ⁽⁶⁾** | **42.7 ±0.8 ⁽⁶⁾** | **40.3 ±1.1 ⁽⁶⁾** | **55.0 ±0.8** | **20.8 ±0.5 ⁽⁶⁾** | **19.9 ±0.6 ⁽⁶⁾** | **18.4 ±0.4 ⁽⁶⁾** | **17.1 ±0.6 ⁽⁶⁾** |
| **CSC** | **1×10⁵** | **7** | **89.2 ±0.7** | **51.7 (*) ±1.9 ⁽⁶⁾** | **49.0 +1.2⁽⁶⁾** | **43.5 ±2.5 ⁽⁶⁾** | **42.0 ±2.5 ⁽⁶⁾** | **54.4 ±1.0** | **23.1 (*) ±1.1 ⁽⁶⁾** | **21.6 ±0.7 ⁽⁶⁾** | **18.7 ±1.3 ⁽⁶⁾** | **18.0 ±1.2 ⁽⁶⁾** |
| **CSC** | **2×10⁶** | **8** | **90.5 ±0.5** | **51.4 (*) ±0.7 ⁽⁷⁾** | **48.4 ±1.3 ⁽⁷⁾** | **45.8 ±1.5 ⁽⁷⁾** | **42.3 ±1.8 ⁽⁷⁾** | **56.3 ±0.8** | **22.9 (*) ±0.4 ⁽⁷⁾** | **21.4 ±0.7 ⁽⁷⁾** | **19.9 ±0.8 ⁽⁷⁾** | **18.1 ±0.9 ⁽⁷⁾** |

### [Heart Weight Measurement]

Eight weeks after the intramyocardial administration of the test or control substance, the animals were euthanized by exsanguination under isoflurane anesthesia following an echocardiographic examination. The hearts were then excised. After removing excess tissue and moisture, the heart weight was measured using an electronic balance (AUX120, Shimadzu Corporation), and the heart-to-body weight ratio was calculated.

As a result, no significant differences in heart weight or heart-to-body weight ratio were observed between the CSC groups (1×10⁵ cells administration and 2×10⁵ cells administration) and the Control group.

## Claims

1. A cell population comprising human-derived cardiac stem cells,
wherein a proportion of cells expressing CD105 among all cells in the cell population is 70% or more,
wherein the population does not express an αMHC gene, and
wherein the population comprises cells with enhanced ADM production ability and/or enhanced MMP1 production ability.

2. The cell population according to claim 1, wherein
the enhanced ADM production ability is demonstrated by an ADM production amount of 1,300 pg/mL or more per 5.0×10⁵ cells after culturing the cell population for 5 days, and/or the enhanced MMP1 production ability is demonstrated by an MMP1 production amount of 100 pg/mL or more per 5.0×10⁵ cells after culturing the cell population for 5 days.

3. The cell population according to claim 1, wherein the population comprises cells with enhanced HGF production ability.

4. The cell population according to claim 3, wherein the enhanced HGF production ability is demonstrated by an HGF production amount of 800 pg/mL or more per 10,000 cells after culturing the cell population for 3 days.

5. The cell population according to claim 3, wherein the enhanced HGF production ability is demonstrated by an HGF production amount of 1,000 pg/mL or more per 10,000 cells after culturing the cell population for 3 days.

6. The cell population according to claim 1, wherein proportions of cells expressing SIRPA, CD45, c-kit, and CD29 among all cells in the population are 60% or more, 5% or less, 30% or less, and 80% or more, respectively.

7. The cell population according to claim 1, wherein proportions of cells expressing SIRPA, CD45, c-kit, and CD29 among all cells in the population are 60% or more, 5% or less, 10% or less, and 80% or more, respectively.

8. The cell population according to claim 1, wherein the cardiac stem cells are derived from human pediatric tissue.

9. A composition comprising the cell population according to any one of claims 1 to 8.

10. A pharmaceutical composition comprising the cell population according to any one of claims 1 to 8.

11. The pharmaceutical composition according to claim 10, for improving cardiac function and/or inhibiting cardiac remodeling.

12. The pharmaceutical composition according to claim 10, for treating cardiac disease.

13. The pharmaceutical composition according to claim 12, wherein the cardiac disease is an ischemic heart disease, dilated cardiomyopathy or hypertrophic cardiomyopathy, or a congenital heart disease including single ventricle disease.

14. The pharmaceutical composition according to claim 10, wherein the composition is an injectable composition.

15. The pharmaceutical composition according to claim 10, wherein the cell population is constructed as a cell tissue in a planar or three-dimensional form.
